# FASCICULE DE BREVET EUROPEEN

(11) **EP 0 642 765 B1**
(45) Date de publication et mention de la délivrance du brevet: **15.12.1999**
(21) Numéro de dépôt: 94420245.6
(22) Date de dépôt: 14.09.1994
(51) Int. Cl.: A61B 17/34

(54) **Trocart pour l'introduction d'instruments d'endoscopie dans des cavités**
Trokar zur Einführung von endoskopischen Instrumenten in Hohlräume
Trocar for introduction of endoscopic instruments in cavities

(30) Priorité: 15.09.1993 FR 9311359
(43) Date de publication de la demande: 15.03.1995
(73) Titulaire: Cuilleron, Jean, F-42100 Saint-Etienne (FR)
(72) Inventeur: Dulucq, Jean Louis, F-33360 Carignan (FR); Cuilleron, Jean, F-42100 Saint Etienne (FR)
(74) Mandataire: Thivillier, Patrick

(56) Documents cités:
- EP-A- 0 538 060
- WO-A-92/14414
- DE-A- 2 845 643
- US-A- 4 112 932
- US-A- 5 112 321
- US-A- 5 211 633
- US-A- 5 234 455

## Description

L'invention se rattache au secteur technique des instruments chirurgicaux.

Dans le cas de certaines interventions chirurgicales, il est connu d'utiliser des trocarts pour permettre l'introduction, dans des cavités du corps humain, de différents types d'instruments d'endoscopie notamment. C'est le cas par exemple, en pneumopéritoine où il est nécessaire de gonfler l'abdomen pour le passage des instruments. Le trocart est conformé pour permettre l'injection de CO2 afin de décomprimer les organes internes.

D'une manière parfaitement connue pour un homme du métier, un trocart comprend, pour l'essentiel, un corps creux présentant en bout, une tête munie d'un canal interne, pour l'introduction de l'instrument considéré. Ce canal interne est en communication avec le corps creux. Pour assurer l'étanchéité lorsque l'on retire l'instrument, afin d'éviter toute perte de CO2 préalablement insuflé, le trocard présente des agencements internes aptes à réaliser cette fonction.

Quels que soient les modes de réalisation des trocarts, ces derniers sont généralement conformés pour ne permettre l'introduction d'instruments que d'un diamètre bien déterminé. Lorsqu'il est nécessaire, en cours d'opération, de changer d'instruments, et si celui-ci a un diamètre supérieur ou inférieur à celui du trocart, le chirurgien se trouve confronté à de réels problèmes.

Pour tenter de remédier à ces inconvénients, on a proposé des trocarts équipés de têtes avec tiroirs coulissants ou disques pivotants afin de correspondre aux différents diamètres des instruments chirurgicaux susceptibles d'être utilisés. On peut citer par exemple, l'enseignement du brevet WO-A-9214414.

Ces solutions ne sont cependant pas satisfaisantes car elles obligent le chirurgien à realiser des manipulations peu rationnelles en utilisant ses deux mains. De plus, ces trocarts ne répondent pas toujours au critère d'étanchéité et sont souvent très encombrants. Enfin, ils sont soit à usage unique, soit restérilisables avec des manipulations de démontage et de nettoyage complexes. Ces inconvénients se retrouvent dans les solutions divulguées par l'enseignement de Brevets EP 0538060 et WO 92/14414.

L'invention s'est fixé pour but de remédier à ces inconvénients d'une manière simple, sûre, efficace et rationnelle.

Les problèmes que se propose de résoudre l'invention sont :
- de pouvoir modifier le diamètre du canal d'admission de la tête du trocart, afin de l'adapter au diamètre de l'instrument désiré dans un appareil compact et ergonomique avec un réducteur de diamètre et un clapet d'étanchéité intégrés dans la tête et manoeuvrables de l'extérieur et d'une seule main, l'autre main pouvant normalement continuer à tenir l'appareil.
- de pouvoir realiser en cours d'intervention l'extraction d'"objets" délicats ou plus encombrants (aiguilles, calculs, etc..) par le même trocart.
- de pouvoir démonter facilement la tête du trocart, partie qui intègre tous les joints et mécanismes difficilement nettoyables.

Pour résoudre de tels problèmes, il a été conçu et mis au point un trocart conforme aux caractéristiques de la revendication 1.

Dans une première forme de réalisation, le réducteur de diamètre est constitué par un bras articulé presentant au moins un trou de diamètre inférieur à celui du canal et apte à être mis en alignement coaxial avec ledit canal, sous l'action du levier.

Pour résoudre le problème posé d'assurer l'étanchéité après introduction de l'instrument, afin d'éviter toute fuite de CO2, le bras est logé transversalement dans un logement formé dans l'epaisseur de la tête, en amont d'un organe d'étanchéité que présente l'entrée du canal de la tête à partir de laquelle est introduit l'instrument. Le ou les trous du bras présentent un organe d'étanchéité coopérant avec le corps de l'instrument correspondant.

Dans une autre forme de realisation, le réducteur de diamètre est constitué par un boisseau sphérique présentant au moins deux trous débouchants de diamètre différent aptes à être mis en alignement coaxial avec le canal sous l'action du lever.

Les deux trous sont en communication et disposés selon deux plans orthogonaux.
Chaque trou présente un organe d'étanchéité coopérant avec le corps de l'instrument correspondant.

Un autre problème que se propose de résoudre l'invention est d'assurer l'auto-pénétration du trocart et sa retenue en sens inverse, tout en ayant pour objectif de positionner correctement le trocart.

Un tel problème est résolu en ce que le corps présente périphériquement sur une partie ou sur la totalité de sa longueur, des cannelures externes annelées.

L'invention est exposée ci-après plus en détail à l'aide des dessins annexés, dans lesquels:
La figure 1 est une vue en coupe longitudinale du trocart selon une première forme de realisation.
La figure 2 est une vue en coupe transversale considérée selon la ligne 2-2 de la figure 1.
La figure 3 est une vue correspondant à la figure 1 montrant le changement de diamètre du canal d'admission.
La figure 4 est une vue en coupe transversale considérée selon la ligne 4-4 de la figure 3.
La figure 5 est une vue en coupe longitudinale du trocart selon une autre forme de realisation et avant introduction d'un instrument.
La figure 6 est une vue en coupe transversale considérée selon la ligne 6-6 de la figure 5.
La figure 7 est une vue en coupe correspondant à la figure 5, montrant le changement de diamètre du canal d'admission et l'introduction de l'instrument sous forme d'un poinçon.
La figure 8 est une vue en coupe transversale considérée selon la ligne 8-8 de la figure 7.

On rappelle pour une meilleure compréhension de la suite de la description, que le trocart comprend un corps (1) équipé d'une tête (2). Le corps se compose d'un tube (1b) surmonté d'une partie évasée (1a). La partie (1b) est percée axialement sur la totalité de sa longueur en (1c), tandis que la tête (2) présente un canal interne débouchant (2a). Le trou (1c) et le canal (2a) sont disposés en alignement coaxial. L'instrument chirurgical devant être utilisé est introduit à partir du canal interne (2a) de la tête (2) pour déborder de l'extrémité libre du corps (1).

La partie évasée (1a) présente un chambrage interne (1d), en communication avec le canal (2a) et le trou (1c). Ce chambrage reçoit un clapet pivotant (3), dont l'axe de rotation se situe au niveau de la tête (2), rappelé en position d'obturation par un ressort (4). En position d'obturation du canal (2a) et du trou (1c), le clapet (3) prend appui sur son siège (2b) comportant un joint d'étanchéité (2c). Ce clapet (3) s'escamote sous l'effet de l'introduction de l'instrument; il peut être également manipulé à partir de l'extérieur par simple pression du doigt sur la manette (3a) lorsque l'opérateur a besoin en cours d'intervention d'extraire de l'organisme des "objets" délicats.

Selon une caractéristique à la base de l'invention, la tête (2) est équipée d'agencements internes manoeuvrables à partir de l'extérieur et aptes à modifier le diamètre du canal (2a) pour le mettre en correspondance avec le diamètre de l'instrument à introduire. Comme il sera indiqué dans la suite de la description, ces agencements sont conformés pour maintenir l'étanchéité à l'intérieur du trocart après introduction de l'instrument.

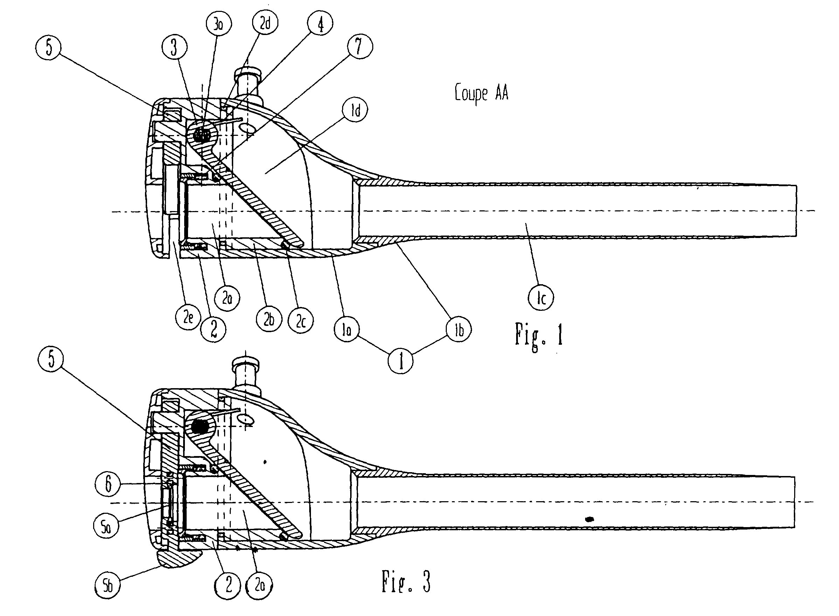

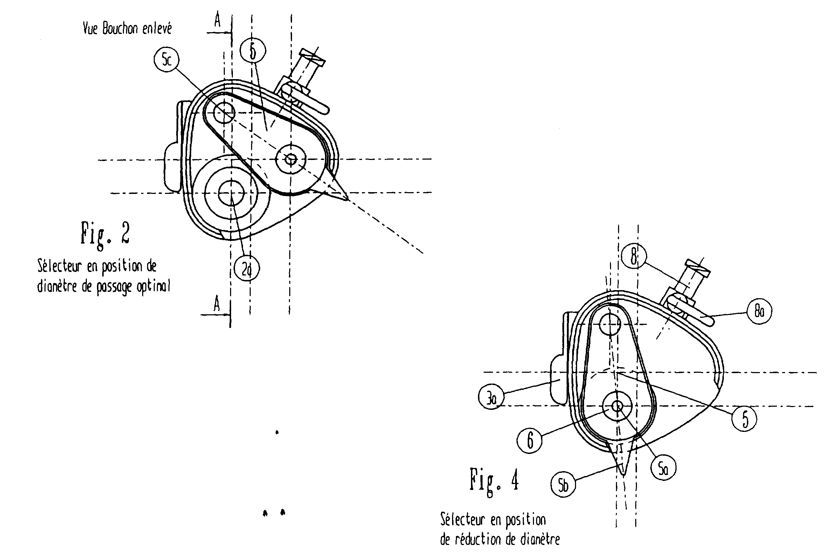

Dans la forme de realisation illustrée figures 1 à 4, les agencements sont constitués par un réducteur de diamètre sous forme d'un bras articulé (5) actionné par un levier (5b). Ce bras (5) présente au moins un trou (5a) de diamètre inférieur à celui du canal (2a) de la tête (2). Ce bras est articulé en (5c) pour être pivoté angulairement lors d'une action exercée sur le levier (5b) afin de mettre en correspondance les trous (5a) et (2a) (figures 3 et 4).

Le réducteur de diamètre (5) est logé transversalement dans un logement (2e) formé dans l'epaisseur de la tête (2). Ce logement (2e) est déterminé pour mettre, en fonction de la position angulaire du levier (5b), soit le trou (5a) en alignement coaxial avec le canal (2a) (figures 3) , soit dégager totalement le canal (2a) (figures 1 et 2). L'axe de rotation (5c) du réducteur de diamètre (5) est parallèle à l'axe du trocart.

Il apparait donc qu'en fonction de la position angulaire du reducteur (5), il est possible de modifier le diamètre d'entrée du canal (2a) pour l'adapter au diamètre de l'instrument à utiliser. Pour assurer l'étanchéité, un joint (7) est monté à l'entrée du canal d'introduction (2a) de la tête (2), en aval du bras articulé (5) et en amont du clapet (3). Ce joint (7) a pour effet d'assurer l'étanchéité de l'instrument dont le diamètre correspond à celui du canal (2a), c'est à dire en position escamotée du réducteur de diamètre (5) (figures 1et 2).

Pour assurer l'étanchéité dans le cas d'un instrument de diamètre plus réduit, c'est à dire lorsque le bras (5) est pivoté pour présenter son trou (5a) en correspondance avec le trou (2a) (figure 3), ledit trou (5a) présente un joint interne (6) apte à coopérer avec le corps de l'instrument correspondant.

A noter que la tête (2), à usage unique, est reliée au corps (1) par un système de encliquetage comportant un joint (2d) assurant l'étanchéité partaite entre les deux parties du trocart.

De plus, pour permettre de regonfler ou de dégonfler le pneumopéritoine, le trocart est équipé d'une vanne (8) ouverte ou fermée par un robinet (8a) quart de tour et cône luer.

Dans une autre forme de realisation, le réducteur de diamètre est constitué par un boisseau sphérique (9) présentant au moins deux trous débouchants (9a) (9b). Ces trous sont de diamètre différent et aptes à être mis en alignement coaxial avec le canal (2a) sous l'action de pivotement angulaire du levier (10).

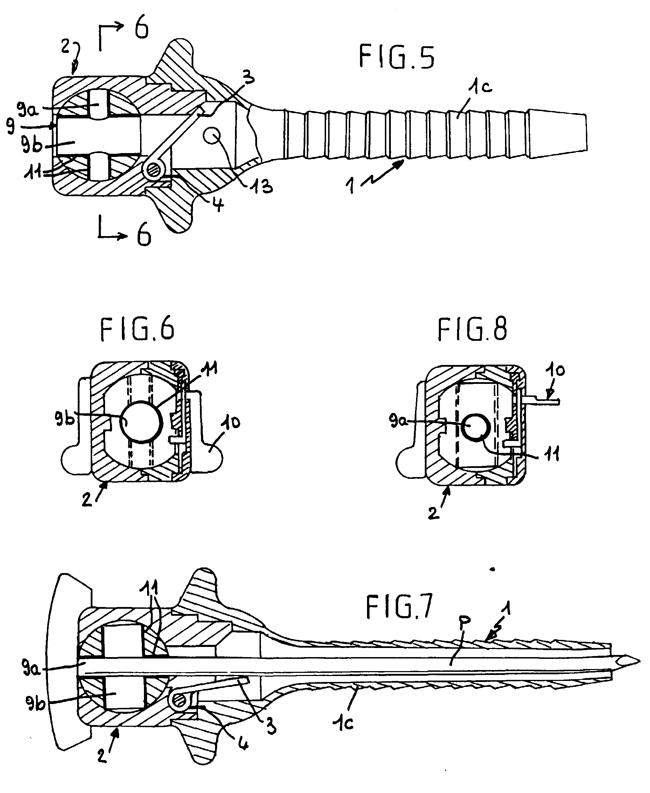

Avantageusement, le boisseau (9) présente deux trous débouchants disposés selon deux plans orthogonaux. Le trou (9a) est de diamètre inférieur au canal (2a) tandis que le trou (9b) est de diamètre égal au diamètre dudit canal. Comme précédemment, afin d'assurer l'étanchéité après introduction de l'instrument, chaque trou (9a) (9b) présente un joint d'étanchéité interne (11). Il suffit donc d'orienter le boisseau en actionnant le levier (10), pour mettre en alignement coaxial avec le canal (2a), soit le trou (9a) (figures 5 et 6), soit le trou (9b) (figures 7 et 8) en fonction du diamètre de l'instrument désiré.

Suivant une autre caractéristique, le corps (1) du trocart présente périphériquement sur une partie ou sur la totalité de sa longueur des cannelures externes annelées (1c).

Les avantages ressortent bien de la description, en particulier on souligne et on rappelle:
- la possibilité de désolidariser facilement la tête et le corps du trocart dans l'optique d'une tête à usage unique,
- la réalisation d'un trocart compact et ergonomique intégrant dans la tête un réducteur de diamètre et un clapet d'étancheité manoeuvrables de l'extérieur et d'une seule main, sans être obligé de lâcher le trocart,
- la possibilité de modifier le diamètre du canal d'introduction de la tête en fonction du diamètre de l'instrument à utiliser,
- la possibilité de réaliser l'extraction d'"objets" délicats en cours d'intervention en escamotant manuellement par un levier extérieur le clapet d'étanchéité,
- l'efficacité du résultat obtenu.

## Revendications

1. Trocart en deux parties du type de ceux présentant une première partie nettoyable et restérilisable sous forme d'un corps creux (1) et une deuxième partie formée par une tête (2) présentant un canal interne débouchant (2a) en alignement coaxial avec ledit corps creux pour l'introduction d'un instrument chirurgical, ledit canal étant assujetti à un moyen d'étanchéité sous forme d'un obturateur à clapet à 45° monté pivotant à l'intérieur de ladite tête,
- la tête (2) étant conçue dans un matériau à usage unique et intègre tous les joints et mécanismes ;
- la tête (2) et le corps (1) étant accouplés par un système d'encliquetage pour être démontables ;
- la tête (2) étant équipée intérieurement d'un réducteur de diamètre (5) ou (9) pivotant par rapport à un axe parallèle à l'axe du trocart et étant actionné par un levier (5b) ou (10) pour mettre en alignement coaxial des trous de différents diamètres avec une partie du canal (2a);
- le levier de commande (5b ou 10) étant apte à être manoeuvré à partir de l'extérieur de la tête par simple pression du doigt.

2. Trocart selon la revendication 1, caractérisé en ce que le réducteur de diamètre est constitué par un bras articulé (5) présentant au moins un trou (5a) de diamètre inférieur à celui du canal (2a), et apte à être mis en alignement coaxial avec ledit canal (2a) sous l'action du lever (5b).

3. Trocart selon la revendication 2, caractérisé en ce que le bras (5) est logé transversalement dans un logement (2e) formé dans l'épaisseur de la tête (2), en amont d'un organe d'étanchéité (7) que présente l'entrée du canal (2a) de la tête (2), à partir de laquelle on introduit l'instrument.

4. Trocart selon la revendication 2, caractérisé en ce que le ou les trous (5a) du bras (5) présentant un organe d'étanchéité coopérant avec le corps de l'instrument correspondant.

5. Trocart selon la revendication 1, caractérisé en ce que le réducteur de diamètre est constitué par un boisseau sphérique (9) présentant au moins deux trous débouchants (9a - 9b) de diamètres différents aptes à être mis en alignement coaxial avec le canal (2a), sous l'action du lever (10).

6. Trocart selon la revendication 5, caractérisé en ce que les deux trous (9a - 9b) sont en communication et disposés selon deux plans orthogonaux.

7. Trocart selon la revendication 6, caractérisé en ce que chaque trou (9a - 9b) présente un organe d'étanchéité coopérant avec le corps de l'instrument correspondant.

8. Trocart selon la revendication 1, caractérisé en ce que le corps (1) présente périphériquement sur une partie ou sur la totalité de sa longueur, des cannelures externes annelées.

## Claims

1. Trocar made up of two parts, of the type having a first cleanable and re-sterilizable part in the form of a hollow body (1), and a second part formed by a head (2) having an internal through-channel (2a) in coaxial alignment with the said hollow body for introduction of a surgical instrument, the said channel being subject to a sealing means in the form of an obturator with a flap valve at 45°, mounted so as to pivot inside the said head,
- the head (2) being made of a disposable material and incorporating all the joints and mechanisms,
- the head (2) and the body (1) being coupled via a locking system so as to be detachable,
- the head (2) being equipped on the inside with a diameter reducer (5) or (9) pivoting with respect to an axis parallel to the axis of the trocar and activated by a lever (5b) or (10) so as to bring holes of different diameters into coaxial alignment with part of the channel (2a),
- the control lever (5b or 10) being able to be manoeuvred from outside the head by simple pressure of the finger.

2. Trocar according to Claim 1, characterized in that the diameter reducer is comprised of an articulated arm (5) having at least one hole (5a) of a diameter less than that of the channel (2a), and able to be brought into coaxial alignment with the said channel (2a) under the action of the lever (5b).

3. Trocar according to Claim 2, characterized in that the arm (5) is lodged transversely in a seat (2e) formed within the thickness of the head (2), upstream of a sealing member (7) present in the inlet of the channel (2a) of the head (2), from which the instrument is introduced.

4. Trocar according to Claim 2, characterized in that the hole or holes (5a) of the arm (5) has or have a sealing member cooperating with the body of the corresponding instrument.

5. Trocar according to Claim 1, characterized in that the diameter reducer is comprised of a spherical bushing (9) having at least two through-holes (9a-9b) of different diameters which can be brought into coaxial alignment with the channel (2a), under the action of the lever (10).

6. Trocar according to Claim 5, characterized in that the two holes (9a-9b) are in communication and are disposed in two orthogonal planes.

7. Trocar according to Claim 6, characterized in that each hole (9a-9b) has a sealing member cooperating with the body of the corresponding instrument.

8. Trocar according to Claim 1, characterized in that the body (1) has on its periphery, along part or all of its length, adjoining external flutings.

## Patentansprüche

1. Zweiteiliger Trokar nach Art der Geräte mit einem ersten reinigungsfähigen und neu sterilisierbaren Teil in Form eines Hohlkörpers (1) und einem zweiten Teil, der aus einem Kopf (2) mit einem in koaxialer Ausrichtung mit dem Hohlkörper verlaufenden ausführenden Innenkanal (2a) für die Einführung eines chirurgischen Instruments gebildet wird, wobei der Kanal von einer Dichtungseinrichtung in Form eines drehbar innerhalb des Kopfes angebrachten 45°-Klappenverschlusses abhängig ist,
wobei der Kopf (2) aus einem Einwegmaterial besteht und alle Dichtungsmitell und Mechanismen umfaßt,
wobei der Kopf (2) und der Körper (1) durch ein Einrastsystem verbunden sind, um demontierbar zu sein,
wobei der Kopf (2) innen mit einem Durchmesserverkleinerer (5) oder (9) ausgestattet ist, der gegenüber einer zur Achse des Trokars parallel verlaufenden Achse drehbar ist und durch einen Hebel (5b) oder (10) betätigt wird, um Löcher verschiedenen Durchmessers mit einem Teil des Kanals (2a) koaxial auszurichten,
wobei der Betätigungshebel (5b oder 10) geeignet ist, durch einfachen Fingerdruck von der Außenseite des Kopfes her manövriert zu werden.

2. Trokar nach Anspruch 1, dadurch gekennzeichnet, daß der Durchmesserverkleinerer aus einem Gelenkarm (5) besteht, der mindestens ein Loch (5a) mit einem geringeren Durchmesser als dem Kanaldurchmesser (2a) aufweist und geeignet ist, bei Betätigung des Hebels (5b) mit dem Kanal (2a) koaxial ausgerichtet zu werden.

3. Trokar nach Anspruch 2, dadurch gekennzeichnet, daß der Arm (5) querliegend in einem Lager (2e) sitzt, das im Vorfeld eines Dichtungsorgans (7) am Eingang des Kanals (2a) des Kopfes (2), von dem aus das Instrument eingeführt wird, in der Materialdicke des Kopfes (2) ausgebildet ist.

4. Trokar nach Anspruch 2, dadurch gekennzeichnet, daß das Loch bzw. die Löcher (5a) des Arms (5) ein Dichtungsorgan aufweisen, das mit dem Körper des entsprechenden Instruments zusammenwirkt.

5. Trokar nach Anspruch 1, dadurch gekennzeichnet, daß der Durchmesserverkleinerer aus einem kugelförmigen Gehäuse (9) besteht, das mindestens zwei ausführende Löcher (9a-9b) unterschiedlichen Durchmessers aufweist, die geeignet sind, bei Betätigung des Hebels (10) koaxial mit dem Kanal (2a) ausgerichtet zu werden.

6. Trokar nach Anspruch 5, dadurch gekennzeichnet, daß die beiden Löcher (9a-9b) miteinander in Verbindung stehen und in zwei orthogonalen Ebenen angeordnet sind.

7. Trokar nach Anspruch 6, dadurch gekennzeichnet, daß jedes Loch (9a-9b) ein Dichtungsorgan aufweist, das mit dem Körper des entsprechenden Instruments zusammenwirkt.

8. Trokar nach Anspruch 1, dadurch gekennzeichnet, daß der Körper (1) umfangsseitig teilweise oder auf ganzer Länge angefügte Außenrillen aufweist.
